(19) **Europäisches Patentamt / European Patent Office / Office européen des brevets**

(11) **EP 4 273 259 B1**

(12) **EUROPEAN PATENT SPECIFICATION**

(45) Date of publication and mention of the grant of the patent:
**05.08.2026 Bulletin 2026/32**

(21) Application number: **21915842.5**

(22) Date of filing: **29.12.2021**

(51) International Patent Classification (IPC):
*C12Q 1/18* (2006.01)   *C12Q 1/02* (2006.01)
*G01N 21/47* (2006.01)   *C12M 1/00* (2006.01)
*C12M 1/34* (2006.01)

(52) Cooperative Patent Classification (CPC):
**C12Q 1/18; G01N 21/4788; G01N 21/253;**
G01N 2021/479

(86) International application number:
**PCT/KR2021/020209**

(87) International publication number:
**WO 2022/146045 (07.07.2022 Gazette 2022/27)**

(54) **ANTIBIOTIC SUSCEPTIBILITY EVALUATION APPARATUS**

VORRICHTUNG ZUR BEWERTUNG DER EMPFINDLICHKEIT GEGENÜBER ANTIBIOTIKA

APPAREIL D'ÉVALUATION DE SENSIBILITÉ AUX ANTIBIOTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2020 KR 20200188357**

(43) Date of publication of application:
**08.11.2023 Bulletin 2023/45**

(73) Proprietor: **The Wave Talk, Inc.**
**Daejeon 34051 (KR)**

(72) Inventors:
• **KIM, Young Dug**
  **Seongnam-si Gyeonggi-do 13597 (KR)**
• **YOO, Jaewoo**
  **Sejong 30153 (KR)**
• **DO, Hyeongkyu**
  **Daejeon 34050 (KR)**
• **CHEON, Doo Young**
  **Uijeongbu-si Gyeonggi-do 11769 (KR)**

(74) Representative: **AWA Sweden AB**
**Box 45086**
**104 30 Stockholm (SE)**

(56) References cited:
WO-A1-2021/006568   WO-A1-99/02645
JP-A- 2016 005 437   KR-A- 20170 132 856
KR-A- 20190 013 541   KR-A- 20210 004 870
KR-B1- 101 686 766   US-A1- 2022 259 636

• HAN SEUNGYUN ET AL: "Rapid antimicrobial susceptibility test using spatiotemporal analysis of laser speckle dynamics of bacterial colonies", INTERNET CITATION, 25 November 2019 (2019-11-25), XP002806196, Retrieved from the Internet <URL:https://www.biorxiv.org/content/10.1101/853168v1> DOI: 10.1101/853168
• G HEJBLUM: "Automated interpretation of disk diffusion antibiotic susceptibility tests with the radial profile analysis algorithm", JOURNAL OF CLINICAL MICROBIOLOGY, vol. 31, no. 9, 1 September 1993 (1993-09-01), US, pages 2396 - 2401, XP093162227, ISSN: 0095-1137, DOI: 10.1128/jcm.31.9.2396-2401.1993
• HAN SEUNGYUN, KIM HYUNJUNG, PARK JONGCHAN, LEE SANGYUN, LEE KYEOREH, KIM JU-KANG, CHUNG HYUN JUNG, PARK YONGKEUN: "Real-time monitoring of bacterial growth and fast antimicrobial susceptibility tests exploiting multiple light scattering.", BIORXIV, 29 November 2018 (2018-11-29), pages 1 - 15, XP055773590, DOI: 10.1101/481184

**EP 4 273 259 B1**

## Description

TECHNICAL FIELD

Embodiments of the present disclosure relate to an evaluation for antibiotics susceptibility.

BACKGROUND ART

A colony counting method and a disk diffusion method are generally used as a method of measuring the antimicrobial activity of a sample. The colony counting method is a method of measuring the antimicrobial activity of the sample in such a manner that a microbial solution having the microorganisms reacted with the sample for a certain time is painted out on a nutrient agar to grow the microorganisms for a certain time and the antimicrobial activity of the sample is measured by using the clustering coefficient of the grown microorganisms, to thereby obtain quantitative measurement results.

In contrast, the disk diffusion method is a method of measuring the size of inhibition zone in such a manner that a specimen is placed on a nutrient agar on which some amounts of the microorganisms are painted out to grow the microorganisms for a certain time and the inhibition zone is generated around the sample due to the sample, to thereby obtain qualitative antimicrobial activity results of the sample.

The colony counting method may obtain the quantitative antimicrobial activity measurement results and is widely used in various research fields, however, requires repeated experiments with various dilution ratios for obtaining a measurable clustering coefficient of the microorganisms and times for reacting with the microorganisms and the sample and for growing the reacted microorganisms on the nutrient agar, respectively, which leads to disadvantages in terms of time and cost.

On the other hand, the disk diffusion method is advantageous in terms of evaluate time and evaluate cost as compared with the colony counting method because a certain amount of microorganisms are painted out on a nutrient agar and a sample is placed on the nutrient agar, and thus, the microorganisms are grown on the nutrient agar together with the sample and the reaction between the microorganisms and the sample takes place with the growth of the microorganism.

However, in such a case, there are also problems in that so as to grow the microorganisms enough to visually check the inhibition zone, the culturing of microorganisms necessarily takes about 16-24 hours and as the diameter of the inhibition zone is manually measured by using a ruler or vernier calipers, the measuring results may vary depending on an inspector.

HAN SEUNGYUN ET AL: "Rapid antimicrobial susceptibility test using spatiotemporal analysis of laser speckle dynamics of bacterial colonies", 25 November 2019 (2019-11-25), DOI: doi:10.1101/853168 discusses that antimicrobial susceptibility testing (AST) is crucial for providing appropriate choices and doses of antibiotics to patients. However, standard ASTs require a time-consuming incubation of about 16-20 h for visual accumulation of bacteria, limiting the use of AST for an early prescription. In this study, they propose a rapid AST based on laser speckle formation (LSF) that enables rapid detection of bacterial growth, with the same sample preparation protocol as in solid-based ASTs.

WO 99/02645 A1 relates to an image analysis system for automated reading of printed multi-character codes, for example on antibiotic susceptibility testing disks, makes use of an orientation means, for example an underline printed beneath the code, to bring the code or its image into canonical alignment with an optimum reading direction for the code. Automated reading of the codes on randomly-orientated AST disks is therefore possible.

G HEJBLUM: "Automated interpretation of disk diffusion antibiotic susceptibility tests with the radial profile analysis algorithm", JOURNAL OF CLINICAL MICROBIOLOGY, vol. 31, no. 9, 1 September 1993 (1993-09-01), pages 2396-2401, ISSN: 0095-1137, DOI: 10.1128/jcm.31.9.2396-2401.1993 discusses that an original algorithm referred to as the radial profile analysis algorithm was implemented on a Macintosh Quadra 700 computer to provide an automatic determination of the inhibition zone diameters of antibiotic susceptibility tests performed with the disk diffusion method.

DETAILED DESCRIPTION OF THE DISCLOSURE

TECHNICAL PROBLEM

An embodiment of the present disclosure provides an antibiotic susceptibility evaluation apparatus for obtaining a sample image and evaluating antibiotic susceptibility more rapidly by deriving a spatial correlation of interference pattern of the sample image.

TECHNICAL SOLUTION TO PROBLEM

The invention is defined by the subject-matter of claim 1.

An embodiment of the present disclosure discloses an antibiotic susceptibility evaluation apparatus including a sample unit including a sample and at least an antibiotic disk arranged on the sample, a light source radiating coherent light to the sample unit, an image sensor detecting transmitted light passing through the sample unit to obtain a sample image, and a controller configured to receive a first sample image, which is an image of the sample at an initial time when the antibiotic disk is arranged on the sample unit, and a second sample image, which is an image of the sample after a preset time, to obtain a spatial correlation of an interference pattern between the first sample image and the second sample image, and to evaluate susceptibility of the sample to antibiotics based on the spatial correlation according a position.

ADVANTAGEOUS EFFECTS OF DISCLOSURE

The antibiotic susceptibility evaluation apparatus according to an embodiment of the present disclosure may evaluate the susceptibility of a sample to antibiotics in a short time without waiting for a sufficient culturing time to generate colonies.

BRIEF DESCRIPTION OF DRAWINGS

FIG.1 is a conceptual view schematically illustrating an antibiotic susceptibility evaluation apparatus according to an embodiment of the present disclosure.
FIG. 2 is a view for explaining elements of the antibiotic susceptibility evaluation apparatus in FIG.1.
FIG. 3 is a view for explaining the sample unit in FIG. 2.
FIGS. 4A and 4B are example views illustrating a measurement screen of an antibiotic susceptibility evaluation apparatus according to an embodiment of the present disclosure.
FIGS. 5 and 6 are views for describing a principle of evaluating the antibiotic susceptibility using the spatial correlation of sample images in the controller.

BEST MODE FOR IMPLEMENTING THE DISCLOSURE

An embodiment of the present disclosure provides an antibiotic susceptibility evaluation apparatus including a sample unit including a sample and at least an antibiotic disk arranged on the sample, a light source radiating coherent light to the sample unit, an image sensor detecting transmitted light passing through the sample unit to obtain a sample image, and a controller configured to receive a first sample image, which is an image of the sample at an initial time when the antibiotic disk is arranged on the sample unit 120, and a second sample image, which is an image of the sample after a preset time, to obtain a spatial correlation of an interference pattern between the first sample image and the second sample image, and to evaluate susceptibility of the sample to antibiotics based on the spatial correlation according a position.

In an embodiment of the present disclosure, the antibiotic susceptibility evaluation apparatus may further include a driving unit rotating the sample unit with respect to a rotation axis, and a plurality of antibiotic disks may be arranged radially with a center at which the rotation axis is positioned.

In an embodiment of the present disclosure, the image sensor may obtain a plurality of sample images of areas around the antibiotic disks while the sample unit is rotated by the driving unit.

In an embodiment of the present disclosure, a center of the light irradiated from the light source may be located at a position spaced apart from the rotational axis.

In the present invention, the controller is configured to calculate a diameter of an inhibition zone of a specified antibiotic disk using a distance to a point at which a resultant value of the spatial correlation corresponds to a preset threshold and to evaluate the susceptibility of the sample to antibiotics using the calculated diameter of the inhibition zone.

In an embodiment of the present disclosure, the controller may be configured to store information on the antibiotic disk, information on the sample, and reference information for evaluating antibiotic susceptibility, to correct the calculated diameter of the inhibition zone by using the information on the antibiotic disk, the information on the sample, and the reference information for evaluating the antibiotic susceptibility, and to evaluate the susceptibility of the sample to antibiotics using the corrected diameter of the inhibition zone.

Other aspects, features and advantages other than the described above will be apparent from the following drawings, claims and detailed description of the present disclosure.

MODE OF DISCLOSURE

Hereinafter, embodiments of the present disclosure are described in detail with reference to the accompanying drawings, and the same reference numerals denote the same or corresponding elements and repeated descriptions on the same elements are omitted when describing with reference to the drawings.

Since the present embodiments may be modified variously, some embodiments are illustrated in the drawings and described in detail in the detailed description. Effects and features of the present disclosure and methods of achieving the same are apparent with reference to embodiments described below in detail with reference to the drawings. However, the present disclosure is not limited to the embodiments disclosed below and may be implemented in various forms.

In the following embodiments, terms such as first and second are used for the purpose of distinguishing one element from another element without limiting meaning.

In the following embodiment, a singular expression includes a plural expression unless it is explicitly meant differently in the context.

In the following embodiments, the term 'include' or 'have' means that a feature or a component described in the specification exists, and the possibility of adding one or more other features or components is not excluded in advance.

In the following embodiment, when a part such as a unit, a region, a component, etc. is on or above another part, it includes not only a case directly on the other part, but also a case where another unit, region, component, etc. is interposed therebetween.

In the following embodiments, the term such as 'connect' and 'couple' do not necessarily mean direct and/or fixed connection or coupling of two members unless the context clearly indicates otherwise, and do not exclude that another member is interposed between the two members.

It means that the features or elements described in the present disclosure exist, and the possibility of adding one or more other features or elements is not excluded in advance.

In the drawings, the size of elements may be exaggerated or reduced for convenience of description. For example, since the size and thickness of each element shown in the drawings are arbitrarily shown for convenience of description, the present disclosure is not necessarily limited to the illustrated.

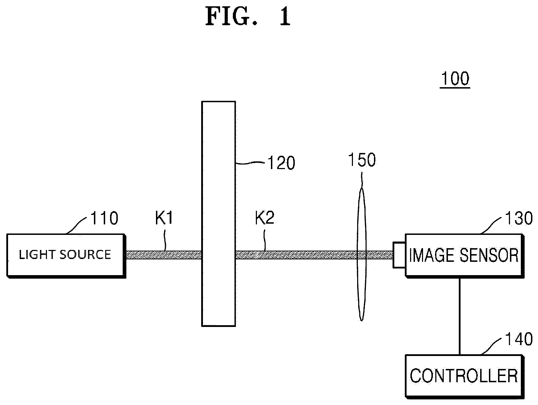

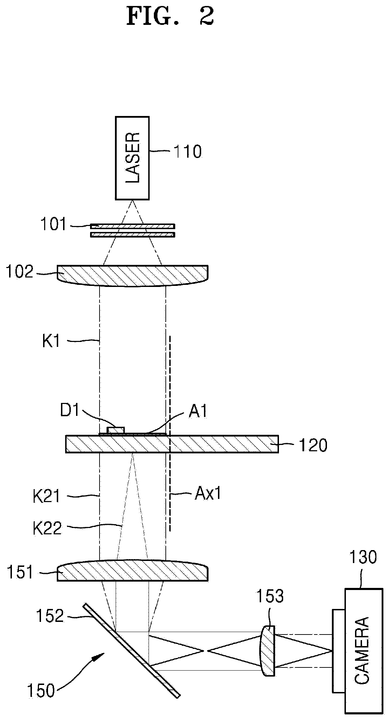

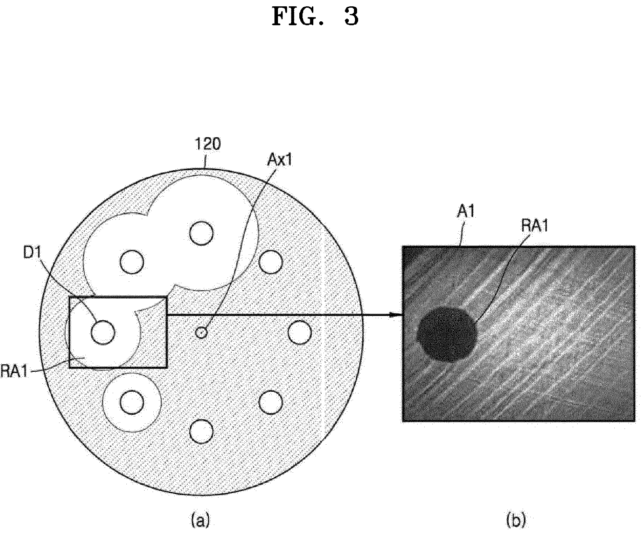

FIG.1 is a conceptual view schematically illustrating an antibiotic susceptibility evaluation apparatus 100 according to an embodiment of the present disclosure, FIG. 2 is a view for explaining elements of the antibiotic susceptibility evaluation apparatus 100 in FIG.1, and FIG. 3 is a view for explaining the sample unit 120 in FIG. 2.

Referring to FIGS.1 to 3, an antibiotic susceptibility evaluation apparatus 100 according to an embodiment of the present disclosure may include a light source 110, a sample unit 120, an image sensor 130, and a controller 140.

The antibiotic susceptibility evaluation apparatus 100 according to embodiments of the present disclosure may be aimed for determining a specific antibiotic that is effective in stopping the growth of a specific bacteria (pathogenic microorganism) which causes infection and may have a technical concept of determining antibiotic susceptibility by applying an antibiotic to a sample containing bacteria and analyzing a sample image obtained by irradiating light to the sample.

The light source 110 may radiate coherent light to the sample unit 120 including the sample. Herein, the light source 110 may include all types of source devices capable of generating light and may be a laser capable of irradiating light of a specific wavelength band.

Herein, the light source 110 may use a laser having good coherence to form a speckle, which is an interference pattern, on the sample. In this case, as the spectral bandwidth of the light source, which determines the coherence of the laser light source, is short, the measurement accuracy may be increased.

That is, as the coherence length increases, the measurement accuracy may increase. Accordingly, a laser light having a spectral bandwidth of the light source 110 less than a predefined reference bandwidth may be used as the light source 110, and the measurement accuracy may increase as the spectral bandwidth of the light source 110 is shorter than the reference bandwidth. For example, the spectral bandwidth of the light source may be set in such a way that the condition of Equation 1 below is satisfied.

[Equation 1]

$$spectral\ bandwidth < 5nm$$

According to Equation 1, when light is irradiated to the sample unit 120 for measuring pattern changes of a laser speckle, the spectral bandwidth of the light source 110 may be maintained to be less than 5 nm.

In addition, when the antibiotic susceptibility evaluation apparatus 100 irradiates the light generated from the light source 110 to the sample unit 120, the characteristic of the light needs to be improved to increase analysis accuracy. To this end, the antibiotic susceptibility evaluation apparatus 100 may include one or more optical means between the light source 110 and the sample unit 120. For example, the optical means may include one or more polarizers 101, collimation lenses 102, etc.

Meanwhile, the light source 110 may be located on a position spaced apart from a central axis Ax1 of the sample unit 120. In other words, the light source 110 may irradiate light to the sample unit 120 in such a way that the light is irradiated onto an area A1 deviated from the center of the sample unit 120.

The sample unit 120 may receive the sample to be tested, and one or more antibiotic disks D1 positioned on the sample may be applied. The sample unit 120 may include any types of containers for receiving the sample, and may be, for example, an agar plate containing a culture solution for culturing microorganisms.

The sample may be a pathogenic microorganism and may be painted out on the agar plate including the culture solution and be received in the sample unit 120. The antibiotic disk D1 may include an antibiotic that is expected to react to the corresponding pathogenic microorganism and may be positioned on the sample unit 120 receiving the sample.

In the sample unit 120, the antibiotic disk D1 may be located at a position spaced a certain distance from the central axis Ax1 of the sample unit 120. The antibiotic disk D1 may be positioned in an area A1 onto which the light source 110 irradiates light.

When the sample unit 120 includes a plurality of antibiotic disks D1, the plurality of antibiotics disks D1 may be radially positioned with respect to the central axis Ax1 of the sample unit 120.

Meanwhile, although not shown in the drawing, the antibiotic susceptibility evaluation apparatus 100 may further include a driving unit (not shown) for rotating the sample unit 120. Herein, since the sample unit 120 may rotate with respect to the central axis Ax1, the central axis Ax1 and the rotation axis are used interchangeably for description hereinafter.

The sample unit 120 may be rotated around the rotation axis Ax1 by the driving unit (not shown). Since the sample unit 120 rotates while the light source 110 and the image sensor 130 may be fixed in a certain position, sample images for a plurality of antibiotic disks D1 may be quickly obtained.

The plurality of antibiotic disks D1 may be arranged on the sample unit 120 in such a way that the antibiotic disks D1 are spaced apart from each other in a radial direction with respect to the rotation axis Ax1 of the sample unit 120, and a gap distance between the neighboring antibiotic disks D1 may be determined in consideration of the size of an inhibition zone RA1. Since the size of the inhibition zone RA1 may be predicted according to a preset reading criterion (e.g., USA: CLSI, Europe: EUCAST), the plurality of antibiotic disks D1 may be arranged to be spaced apart from each other based on the size of the inhibition zone RA1.

However, the antibiotic disks D1 may not be necessarily spaced apart by the maximal size of the inhibition zone, and thus, may be arranged in consideration of partial overlaps. As shown in the drawings, this is because the antibiotic disks D1 do not overlap in the radial direction that is directed from the antibiotic disk D1 to the rotation axis Ax1 even though the antibiotic disks D1 are partially overlapped in an arrangement direction.

The image sensor 130 may be positioned on a path of light emitted from the sample unit 120, and thus, obtain a sample image which is an optical image photographed by light. The image sensor 130 may detect transmitted light K2 passing through the sample unit 120, to thereby obtain the sample image. For example, the image sensor 130 may be a charge-coupled device (CCD) camera. The image sensor 130 may measure the optical image photographed by light emitted from the sample unit 120 and transmit the optical image to the controller 140.

The antibiotic susceptibility evaluation apparatus 100 according to an embodiment of the present disclosure may further include a lens unit 150 between the image sensor 130 and the sample unit 120. Herein, the lens unit 150 may include one or more lenses 151 and 153 and may further include an optical component such as a mirror 152.

The antibiotic susceptibility evaluation apparatus 100 may implement an effect of shortening a measurement distance z between the image sensor 130 and the sample unit 120 by using the lens unit 150, to thereby perform an early detection.

At this time, assuming that the lens unit 150 includes a single lens, the focal distance f of the lens is expressed as $ab/(a+b)$ and the magnification M of the lens is expressed as $a/b$, where a is a distance between the lens and the image sensor 130 and b is a distance between the lens and a virtual image of the image sensor 130.

For example, assuming that a width of the observation area A1 for observing the sample is greater than 30 mm and the size (radius, w) of colony that is observable by the present evaluation apparatus 100 is greater than 20 $\mu$m (based on coliform bacillus (E. coli) culturing for 4 hours), a pixel size p of the image sensor 130 may have the following relational expression based on the Nyquist-shannon Sampling theorem.

【Equation 2】

$$\frac{2p}{M} < w$$

Meanwhile, the antibiotic susceptibility evaluation apparatus 100 may determine a propagation distance z that is a distance from the sample unit 120 to the virtual image of the image sensor 130. At this time, assuming that the sample is a thin and transparent disk, the sample to be measured may be characterized by a radius w that is a size of the sample, a thickness h, and a refractive index n. Since the propagation distance z is much longer than the size of the sample, the Fraunhofer approximation may be applied.

Herein, the final interference pattern may appear as an interference pattern between an object wave K22, which is a diffraction pattern by the sample, and a reference wave K21 transmitted after irradiating from the light source 110 to the sample unit 120 and may be shown in an airy pattern.

For the above conditions, the intensity of the interference pattern is expressed on a polar coordinate system as follows.

【Equation 3】

$$I(r) = \left| e^{jkz} e^{j\frac{kr^2}{2z}} \frac{\pi w^2}{j\lambda z} \left[ 2\frac{J_1(kwr/z)}{kwr/z} \right] + e^{jkhn} \right|^2$$

At this time, since $\left[ 2\frac{J_1(kwr/z)}{kwr/z} \right]$ has a maximum value of 1 when r equals to 0, the above equation is expressed again as follows.

【Equation 4】

$$\begin{aligned} I(0) &= \left| e^{jkz} \frac{\pi w^2}{j\lambda z} + e^{jkhn} \right|^2 \\ &= \left| \left( \cos(khn) + \frac{\pi w^2}{\lambda z}\sin(kz) \right) + j\left( \sin(khn) - \frac{\pi w^2}{\lambda z}\cos(kz) \right) \right|^2 \\ &= 1 + \frac{\pi^2 w^4}{\lambda^2 z^2} + \frac{2\pi w^2}{\lambda z}\sin(k(z - hn)) \end{aligned}$$

Equation 4 shows that the signal intensity decreases as the propagation distance z increases, and the signal intensity increases as the sample size w increases. When the thickness h of the sample is almost equal to the wavelength $\lambda$ (about 0.5 $\mu$m), I(r) becomes a maximal value when r equals to zero. In general, since an analog signal noise ratio (SNR) of 6dB is converted to 1-bit when being digitized, the propagation distance z may be determined as a distance satisfying a condition in which the maximal value of I(r) is greater than the 6dB SNR of the image sensor 130.

The controller 140 may evaluate the antibiotic susceptibility of the sample using the sample image (see (b) in FIG. 3) obtained by the image sensor 130. Specifically, the controller 140 may receive a first sample image that is an initial image when the antibiotic disk D1 is positioned on the sample unit 120 and a second sample image that is an image after a preset time passes, obtain the spatial correlation of the interference pattern between the first sample image and the second sample image, and evaluate the susceptibility of the sample to the antibiotic based on the spatial correlation according to a position. Detailed descriptions on the antibiotic susceptibility evaluate using the spatial correlation in the controller 140 are described below.

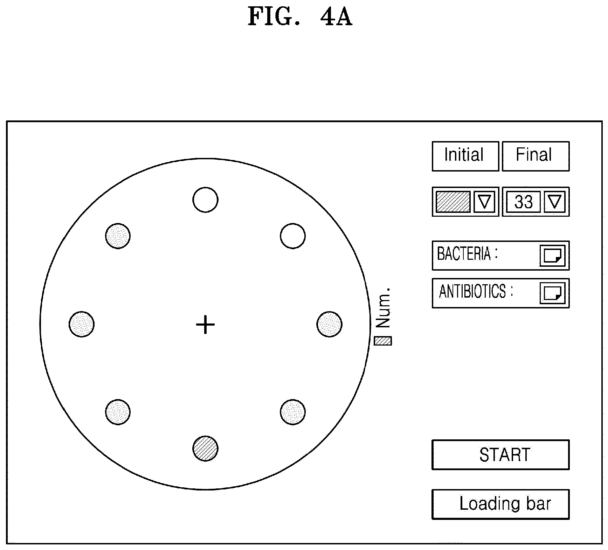

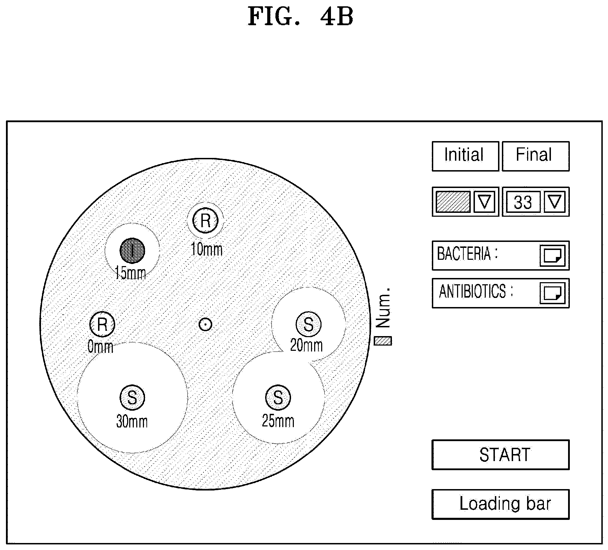

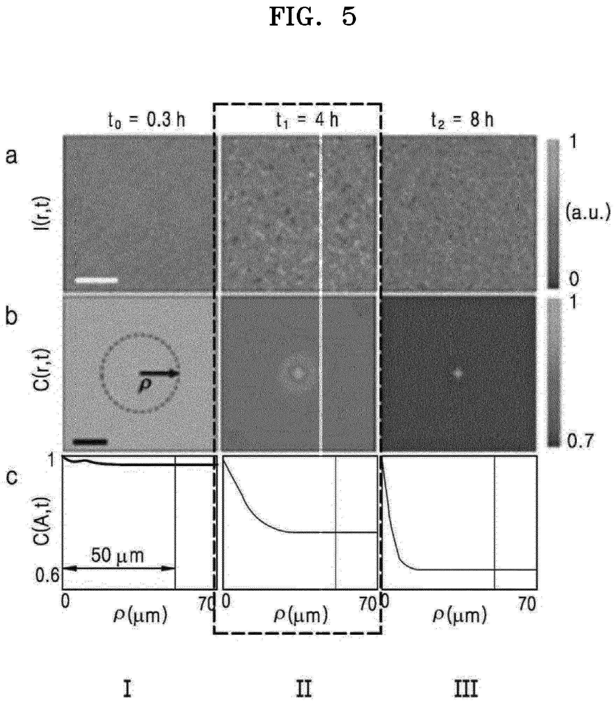

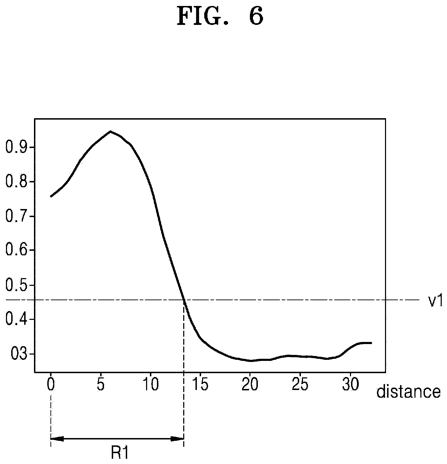

FIGS. 4A and 4B are example views illustrating a measurement screen of the antibiotic susceptibility evaluation apparatus 100 according to an embodiment of the present disclosure, and FIGS. 5 and 6 are views for describing a principle of evaluating the antibiotic susceptibility using the spatial correlation of sample images in the controller.

Referring to FIG. 4A, the antibiotic susceptibility evaluation apparatus 100 may further include an input/output interface, for example, a touch display unit (not shown). The display unit (not shown) may obtain information on the samples from a user and provide measurement results to the user.

At this time, the display unit (not shown) may display the sample image captured by the image sensor 130 on the screen. However, as described above, the sample image may be an image obtained by measuring only a certain area including the antibiotic disk D1 on the sample unit 120. In another embodiment, the display unit (not shown) may display a sample unit image obtained by capturing the entire sample unit 120. To this end, the antibiotic susceptibility evaluation apparatus 100 may further include a camera for capturing an entire image of the sample unit 120 as well as the image sensor 130.

In an embodiment, the antibiotic susceptibility evaluation apparatus 100 may receive information on the sample and information on the antibiotic disk applied to the sample by the input/output interface. In addition, the antibiotic susceptibility evaluation apparatus 100 may receive reference information for evaluating the antibiotic susceptibility. For example, the reference information for the antibiotic susceptibility evaluate may be a preset reading criterion (e.g., USA:

CLSI, Europe: EUCAST).

When the user prepares the sample unit 120 including the sample and the antibiotic disk in the antibiotic susceptibility evaluation apparatus 100, the antibiotic evaluation apparatus 100 may obtain the first sample image of the sample unit 120 at first. The antibiotic susceptibility evaluation apparatus 100 may obtain the initial first sample image as a single image or a plurality of images that are sequentially captured in time order.

Referring to FIG. 4B, the antibiotic susceptibility evaluation apparatus 100 may obtain the second sample image after a preset time passes. Herein, the preset time is a growing time in which the sample grows enough to derive meaningful results in the antibiotic susceptibility evaluation apparatus 100, and may be, for example, about 4 to 6 hours. However, the present disclosure is not limited thereto, and the preset time may vary depending on the type of sample, and the time may be reduced according to development. However, the preset time disclosed above may be much shorter than the time required for visual confirmation by the conventional disk diffusion method (e.g., about 16 to 24 hours), and the antibiotic susceptibility evaluation apparatus 100 according to an embodiment of the present disclosure may have an advantage that the antibiotic susceptibility evaluate is performed in a shorter time compared to conventional techniques.

When the preset time has passed, as shown in FIG. 4B, the inhibition zones RA1 may be formed in the sample by the antibiotic disks D1. The sizes of the inhibition zones RA1 may vary depending on the type of sample and the type of antibiotics.

A plurality of antibiotic disks D1 may be applied to the sample unit 120, and, so as to evaluate the susceptibility from each of the antibiotic disks D1, the antibiotic susceptibility evaluation apparatus 100 may rotate the sample unit 120 and capture the second image for each of the antibiotic disks D1. The same method as described above may be applied when obtaining the first sample image at first time.

Referring to FIGS. 5 and 6, the controller 140 may receive the first sample image that is an image at an initial time when the antibiotic disks D1 are arranged on the sample unit 120 and the second sample image after the preset time passes from the image sensor 130. The controller 140 may obtain the spatial correlation of the interference patterns between the first sample image and the second sample image and evaluate the susceptibility of the sample to the antibiotic based on the spatial correlation according to a position.

Specifically, when the colony is not sufficiently grown, most of the light that is incident on the sample unit 120 is emitted out of the sample unit because there is no diffusion medium, but when the colony sufficiently grows, the light that is incident on the sample unit 120 is scattered from the colonies to thereby form the interference pattern. At this time, when the antibiotic disks D1 are applied to the sample, the interference pattern may be greatly changed at the boundaries of the inhibition zones RA1 that are formed by the antibiotics.

The controller 140 may obtain the spatial correlation of the interference pattern. Herein, the spatial correlation given by the following equation may be expressed as a number in a certain range that indicates how much brightness of an arbitrary pixel is similar to that of a pixel spaced apart from the arbitrary pixel by a distance r on an image captured at time t.

The certain range may be in a range of -1 to 1. That is, the spatial correlation indicates a degree of correlation between an arbitrary pixel and another pixel, and 1 indicates a positive correlation, -1 indicates a negative correlation, and 0 indicates no correlation. Specifically, since the brightness is emitted uniformly before the interference pattern is formed, the spatial correlation of the sample image shows a positive correlation close to 1, but after the interference pattern is formed, the value of the correlation may decrease in a direction close to 0.

In the image sensor 130, the brightness of a pixel at the position of $r' = (x, y)$ detected at time t may be defined as $I(r', t)$, and the brightness of the pixel spaced apart by r may be defined as $I(r' + r, t)$. Based on the definition described above, the spatial correlation may be expressed as following Equation 5.

## [Equation 5]

$$C(r, t) = \frac{1}{C_0(t)} \iint I(r' + r, t) I(r', t) dr'$$

$C_0(t)$ is used to adjust Equation 7 in a range of -1 to 1. When the brightness $I(r', t)$ of an arbitrary pixel at time t is the same as the brightness $I(r'+r, t)$ of a pixel spaced apart from the arbitrary pixel by a distance r, the spatial correlation may be derived by 1, otherwise the spatial correlation may have a value less than 1.

In an embodiment, the present disclosure may express the above-mentioned spatial correlation only as a function of time. Thus, the controller 140 may obtain an average of the spatial correlations for various pixels spaced apart from an arbitrary pixel by the same distance r by using Equation 6 below (see b in FIG. 5).

【Equation 6】

$$C(\rho, \mathrm{t}) = \frac{1}{2\pi} \int_0^{2\pi} C(r,t)\,d\theta$$

In an embodiment, the controller 140 may substitute the variable r in Equation 6 with a preset distance to make Equation 6 a function of time, and then, may check the degree of interference pattern formation as a value in a certain range of 0 and 1 by using the function.

The controller 140 may distinguish the inhibition zones RA1 from the sample image by a pattern change of the sample image over time. Specifically, the controller 140 may distinguish edge areas of the inhibition zones RA1 by the pattern change of the sample image over time.

Two identical superimposed images may be generated by using a single image and one of the two identical images may be shifted by a preset distance in a direction, and then, it is analyzed how much a pair of pixels, which are adjacent to each other in the shifted image and an unshifted image, are similar to each other, to thereby obtain the spatial correlation. Herein, the spatial correlation may function as a criterion for indicating how uniform the images are, and thus, when the interference pattern is generated by the colonies, the similarity of two adjacent pixels decreases due to the shabby interference pattern and the value of the spatial correlation is also decreased.

The spatial correlation coefficient may vary according to the shifted distance r, and more particularly, the spatial correlation coefficient decreases as the shifted distance r increases in a certain distance range and when the shifted distance r exceeds the certain distance range, the spatial correlation coefficient has a substantially constant value. Accordingly, in order to obtain a more meaningful spatial correlation, the controller 140 may obtain the spatial correlation from images that are shifted from each other over a preset distance. At this time, the preset distance r may depend on the speckle size, and the controller 140 may obtain the spatial correlation by shifting the image by a distance corresponding to some pixels larger than the speckle in a pixel unit. For example, the preset distance may be a distance of at least 3 pixels or more.

When the sample includes microorganisms growing with time, the concentration of the microorganism increases as time passes and the spatial correlation coefficient may be changed more greatly even when the microorganisms are shifted by the same distance r. Therefore, the antibiotic susceptibility evaluation apparatus 100 according to an embodiment of the present disclosure may quickly evaluate the antibiotic susceptibility due to the analysis of the spatial correlation coefficient without waiting for the culturing time until which the microorganisms are cultured and formed into visually shown colonies.

As shown in FIG. 6, the controller 140 may obtain the spatial correlation coefficient in relation with the distance by the same method as described above and evaluate the susceptibility of the sample to the antibiotic by using the spatial correlation coefficient. In the invention, the controller 140 calculates diameters of the inhibition zones RA1 of the corresponding antibiotic disks D1 by using a distance R1 to a point corresponding to the preset threshold value v1 and evaluate the susceptibility of the sample to antibiotics by using the calculated diameters of the inhibition zones RA1.

The threshold value v1 is a preset value and may function as a reference value for determining a point where the spatial correlation coefficient rapidly changes as edge areas of the inhibition zones RA1. The threshold value v1 may vary depending on the type of antibiotics or the sample and may also have different values depending on time.

At this time, the controller 140 may correct the diameters of the calculated inhibition zones RA1 by using the information on the antibiotic disks D1, the information on the samples, and the reference information for evaluating the antibiotic susceptibility. In other words, when a specific antibiotic is applied to a sample that is known in advance, the size of the inhibition zone is to be identical or similar to the reference information for evaluating the antibiotic susceptibility However, when the reference information is applied, since there are many cases that the reference information is determined with the naked eye, the size of the inhibition zone may be slightly different from the actual measuring result.

When the diameters of the inhibition zones RA1 are different from the reference information, the controller 140 may correct the diameters of the inhibition zones RA1 based on the reference information. The controller 140 may receive and store the reference information additionally.

In another embodiment, the controller 140 may learn reference information for classifying the size of the inhibition zone based on the information on the antibiotic disks D1 and the information on the sample. The controller 140 applies the information on the antibiotic disk D1 and the information on the sample to a neural network model to learn the reference information for classifying the sizes of the inhibition zones RA1, and automatically correct the size of the inhibition zones RA1 according to the reference information.

The neural network model may be learned using any one of deep neural networks (DNN), convolutional neural networks (CNN), recurrent neural network (RNN), and deep belief networks (DBN).

The controller 140 may derive the sizes of the inhibition zones RA1 using the learned reference information and evaluate the susceptibility of the sample to antibiotics using the derived data of the inhibition zones RA1.

The controller 140 may be a processor configured to process commands of a computer program by performing basic arithmetic, logic, and input/output operations. The command may be provided to the controller 140 by a memory or a communication unit. For example, the controller 140 may be configured to execute the received commands according to program codes stored in a recording device such as a memory. Herein, the term 'processor' may include, for example, a data processing device embedded in a hardware having a physically structured circuit for performing a function represented by a code or a command in a program.

An example of the data processing device embedded in the hardware may include a microprocessor, a central processing unit (CPU), a processor core, a multiprocessor, an application-specific integrated circuit (ASIC), a field programmable gate array (FPGA), etc., but is not limited thereto.

As described above, the antibiotic susceptibility evaluation apparatus according to embodiments of the present disclosure may evaluate the susceptibility of a sample to antibiotics in a short time without waiting for the culturing time when the colonies are sufficiently generated.

So far, a preferred embodiment of the present disclosure has been described. Those skilled in the art to which the present disclosure belongs will understand that the present disclosure may be implemented in a modified form without departing from the essential characteristics of the present disclosure. Therefore, the disclosed embodiments should be considered from a descriptive point of view rather than a limiting point of view. The scope of the present disclosure is shown in the claims rather than the above description, and all differences within the equivalent scope should be construed as being included in the present disclosure.

INDUSTRIAL APPLICABILITY

According to an embodiment of the present disclosure, an antibiotic susceptibility evaluation apparatus is provided. In addition, the embodiments of the present disclosure may be applied to a microorganism detection device that is used industrially.

**Claims**

1. An antibiotic susceptibility evaluation apparatus (100) comprising:

   a sample unit (120) accommodating a sample and one or more antibiotic disks (D1) arranged on the sample;
   a light source (110) radiating coherent light to the sample unit (120);
   an image sensor (130) detecting transmitted light passing through the sample unit (120) to obtain a sample image; and
   a controller (140) configured to:

   receive a first sample image at an initial time when the one or more antibiotic disks (D1) are arranged on the sample unit (120), and a second sample image after a preset time;
   obtain a spatial correlation of interference patterns of the first sample image and the second sample image; and
   evaluate susceptibility of the sample to antibiotics based on the spatial correlation according to a position,

   wherein the controller (140) is further configured to:

   calculate a diameter of an inhibition zone (RA1) of a corresponding antibiotic disk (D1) using a distance (R1) to a point at which a resultant value of the spatial correlation of the second sample image corresponds to a preset threshold (v1), and
   evaluate the susceptibility of the sample to antibiotics using the calculated diameter of the inhibition zone (RA1).

2. The antibiotic susceptibility evaluation apparatus of claim 1, further comprising

   a driving unit rotating the sample unit with respect to a rotation axis,
   wherein when the number of the one or more antibiotic disks is 2 or more, the one or more antibiotic disks are arranged radially with a center at which the rotation axis is positioned.

3. The antibiotic susceptibility evaluation apparatus of claim 2, wherein
   the image sensor obtains a sample image of a certain area in which the one or more antibiotic disks are positioned while the sample unit is rotated by the driving unit.

4. The antibiotic susceptibility evaluation apparatus of claim 2, wherein
   a center of the coherent light irradiated from the light source is located at a position spaced apart from the rotational axis.

5. The antibiotic susceptibility evaluation apparatus of claim 1, wherein
   the controller is configured to:

   store information on the one or more antibiotic disks, information on the sample, and reference information for evaluating antibiotic susceptibility;
   correct the calculated diameter of the inhibition zone by using the information on the one or more antibiotic disks, the information on the sample, and the reference information for evaluating the antibiotic susceptibility; and
   evaluate the susceptibility of the sample to antibiotics using the corrected diameter of the inhibition zone.

## Patentansprüche

1. Vorrichtung zur Bestimmung der Antibiotikaempfindlichkeit (100), umfassend:

   eine Probeneinheit (120), die eine Probe und eine oder mehrere auf der Probe angeordnete Antibiotika-Scheiben (D1) aufnimmt;
   eine Lichtquelle (110), die kohärentes Licht auf die Probeneinheit (120) strahlt;
   einen Bildsensor (130), der das durch die Probeneinheit (120) hindurchtretende Durchlicht erfasst, um ein Probenbild zu erhalten; und
   eine Steuerung (140), die so konfiguriert ist, dass:
   ein erstes Probenbild zu einem Anfangszeitpunkt, wenn die eine oder mehrere Antibiotika-Scheiben (D1) auf der Probeneinheit (120) angeordnet sind, und ein zweites Probenbild nach einer voreingestellten Zeit empfangen wird;
   eine räumliche Korrelation der Interferenzmuster des ersten Probenbilds und des zweiten Probenbilds ermittelt wird; und
   die Empfindlichkeit der Probe gegenüber Antibiotika basierend auf der räumlichen Korrelation entsprechend einer Position bestimmt wird,
   wobei die Steuerung (140) ferner so ausgelegt ist, dass sie:

   einen Durchmesser einer Hemmzone (RA1) einer entsprechenden Antibiotika-Scheibe (D1) anhand eines Abstands (R1) zu einem Punkt berechnet, an dem ein resultierender Wert der räumlichen Korrelation des zweiten Probenbildes einem voreingestellten Schwellenwert (v1) entspricht, und
   die Empfindlichkeit der Probe gegenüber Antibiotika anhand des errechneten Durchmessers der Hemmzone (RA1) bestimmt wird.

2. Vorrichtung zur Bewertung der Antibiotikaempfindlichkeit nach Anspruch 1, ferner umfassend

   eine Antriebseinheit, die die Probeneinheit um eine Drehachse dreht,
   wobei, wenn die Anzahl der einen oder mehreren Antibiotika-Scheiben 2 oder mehr beträgt, die eine oder mehreren Antibiotika-Scheiben radial um ein Zentrum angeordnet sind, an dem sich die Drehachse befindet.

3. Vorrichtung zur Bestimmung der Antibiotikaempfindlichkeit nach Anspruch 2, wobei
   der Bildsensor ein Probenbild eines bestimmten Bereichs erhält, in welchem sich die eine oder mehreren Antibiotika-Scheiben befinden, während die Probeneinheit durch die Antriebseinheit gedreht wird.

4. Vorrichtung zur Bestimmung der Antibiotikaempfindlichkeit nach Anspruch 2, wobei
   ein Mittelpunkt des von der Lichtquelle ausgestrahlten kohärenten Lichts sich an einer von der Drehachse beabstandeten Stelle befindet.

5. Vorrichtung zur Bestimmung der Antibiotikaempfindlichkeit nach Anspruch 1, wobei

   die Steuerung so konfiguriert ist, dass sie:

Informationen zu der einen oder den mehreren Antibiotika-Scheiben, Informationen zu der Probe sowie Referenzinformationen zur Bestimmung der Antibiotikaempfindlichkeit speichert;
den errechneten Durchmesser der Hemmzone unter Verwendung der Informationen zu der einen oder den mehreren Antibiotika-Scheiben, der Informationen zu der Probe sowie der Referenzinformationen zur Bestimmung der Antibiotikaempfindlichkeit korrigiert; und
die Empfindlichkeit der Probe gegenüber Antibiotika anhand des korrigierten Durchmessers der Hemmzone bestimmt.

**Revendications**

1. Appareil d'évaluation de la sensibilité aux antibiotiques (100) comprenant :

   une unité d'échantillon (120) recevant un échantillon et un ou plusieurs disques d'antibiotiques (D1) disposés sur l'échantillon ;
   une source de lumière (110) émettant une lumière cohérente vers l'unité d'échantillon (120) ;
   un capteur d'image (130) détectant la lumière transmise traversant l'unité d'échantillonnage (120) afin d'obtenir une image de l'échantillon ; et
   un contrôleur (140) configuré pour :

      recevoir une première image de l'échantillon à un instant initial lorsque le ou les disques d'antibiotiques (D1) sont disposés sur l'unité d'échantillonnage (120), et une seconde image de l'échantillon après un temps prédéfini ;
      obtenir une corrélation spatiale des motifs d'interférence de la première image de l'échantillon et de la seconde image de l'échantillon ; et
      évaluer la sensibilité de l'échantillon aux antibiotiques sur la base de la corrélation spatiale en fonction d'une position,
      dans lequel le contrôleur (140) est en outre configuré pour :

         calculer un diamètre d'une zone d'inhibition (RA1) d'un disque antibiotique correspondant (D1) à l'aide d'une distance (R1) par rapport à un point auquel une valeur résultante de la corrélation spatiale de la seconde image d'échantillon correspond à un seuil prédéfini (v1), et
         évaluer la sensibilité de l'échantillon aux antibiotiques à l'aide du diamètre calculé de la zone d'inhibition (RA1).

2. Appareil d'évaluation de la sensibilité aux antibiotiques selon la revendication 1, comprenant

   en outre une unité d'entraînement faisant tourner l'unité d'échantillon par rapport à un axe de rotation,
   dans lequel, lorsque le nombre du ou des disques d'antibiotiques est supérieur ou égal à 2, le ou les disques d'antibiotiques sont disposés de manière radiale autour d'un centre au niveau duquel est positionné l'axe de rotation.

3. Appareil d'évaluation de la sensibilité aux antibiotiques selon la revendication 2, dans lequel
   le capteur d'image acquiert une image de l'échantillon d'une certaine zone dans laquelle le ou les disques d'antibiotiques sont positionnés tandis que l'unité d'échantillon est mise en rotation à l'aide de l'unité d'entraînement.

4. Appareil d'évaluation de la sensibilité aux antibiotiques selon la revendication 2, dans lequel
   un centre de la lumière cohérente émise par la source de lumière est situé à une position espacée de l'axe de rotation.

5. Appareil d'évaluation de la sensibilité aux antibiotiques selon la revendication 1, dans lequel le contrôleur est configuré pour :

   stocker des informations sur le ou les disques d'antibiotiques, des informations sur l'échantillon et des informations de référence pour l'évaluation de la sensibilité aux antibiotiques ;
   corriger le diamètre calculé de la zone d'inhibition à l'aide des informations sur le ou les disques d'antibiotiques, des informations sur l'échantillon et des informations de référence pour l'évaluation de la sensibilité aux antibiotiques ; et
   évaluer la sensibilité de l'échantillon aux antibiotiques à l'aide du diamètre corrigé de la zone d'inhibition.

# FIG. 1

# FIG. 2

# FIG. 3

(a)                                                        (b)

# FIG. 4A

# FIG. 4B

# FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9902645 A1

**Non-patent literature cited in the description**

- **HAN SEUNGYUN et al.** *Rapid antimicrobial susceptibility test using spatiotemporal analysis of laser speckle dynamics of bacterial colonies*, 25 November 2019

- **G HEJBLUM**. Automated interpretation of disk diffusion antibiotic susceptibility tests with the radial profile analysis algorithm. *JOURNAL OF CLINICAL MICROBIOLOGY*, 01 September 1993, vol. 31, ISSN 0095-1137, 2396-2401